# DEMANDE DE BREVET EUROPEEN

(11) **EP 1 604 638 A2**
(43) Date de publication de la demande: **14.12.2005**
(21) Numéro de dépôt: 05291042.9
(22) Date de dépôt: 13.05.2005
(51) Int. Cl.: A61K 7/027, A61K 7/02, A61K 7/48, A61K 7/021

(54) **Composition cosmétique comprenant un polymére semi-cristallin et un ester de dimère diol**

(30) Priorité: 08.06.2004 FR 0406168
(71) Demandeur: L'OREAL, 75008 Paris (FR)
(72) Inventeur: Lebre, Caroline, 94320 Thiais (FR); Ferrari, Véronique, 94700 Maisons-Alfort (FR)
(74) Mandataire: Chantraine, Sylvie Hélène

(57) **Abrégé**

La présente invention a pour objet une composition cosmétique comprenant au moins un ester de dimère diol et d'au moins un acide mono-carboxylique en C₄ à C₃₄ ou di-carboxylique, et au moins un polymère semi-cristallin à structure organique dont la température de fusion est supérieure ou égale à 30°C.

## Description

La présente invention se rapporte à une composition cosmétique, notamment une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable.

La composition de l'invention peut en particulier constituer un produit de maquillage et/ou de soin de la peau et/ou des lèvres ; du corps, ou des phanères d'êtres humains ayant en particulier des propriétés de soin et/ou de traitement n on thérapeutique. Elle constitue notamment un rouge à lèvres ou un brillant à lèvres, une base ou baume de soin pour les lèvres, un fond de teint, un fard à joues ou à paupières, un produit pour tatouage, un mascara, un produit anti-cernes, un eye-liner, un vernis à ongles, un produit de bronzage artificiel de la peau, un produit de coloration ou de soin des cheveux.

Il existe de nombreuses compositions cosmétiques pour lesquelles les propriétés de brillance du film déposé, après application sur les matières kératiniques (peau, lèvres, phanères), sont souhaitables. On peut citer par exemple les rouges à lèvres, les vernis à ongles ou encore certains produits capillaires. Pour ce faire, le formulateur utilise généralement à titre d'agent actif principal en terme de brillance des lanolines associées à au moins une huile dite « brillante », comme a) des polymères huileux tels que les polybutènes qui ont une viscosité élevée, b) des esters d'acide ou d'alcool gras dont le nombre de carbone est élevé (typiquement supérieur à 16), ou encore c) certaines huiles végétales. Toutefois, les compositions de l'art antérieur brillantes présentent le désavantage d'avoir une tenue insuffisante au cours du temps, en particulier de migrer hors du tracé initial du maquillage.

On recherche donc des compositions cosmétiques de maquillage ou de soin formant un dépôt présentant une bonne tenue au contact des liquides mis en contact avec le maquillage, notamment au cours d'un repas, et ne migrant pas.

L'invention a précisément pour objet de proposer une association particulière comprenant au moins un ester de dimère diol avec un diacide dicarboxylique spécifique permettant avantageusement de donner satisfaction en terme de brillance tout en améliorant la non migration de la composition cosmétique.

Les esters de dimères diols et d'acides mono ou dicarboxyliques ont été décrits d'une manière générale dans le document FR 2 795 309 comme étant utiles pour préparer des compositions cosmétiques dotées notamment de propriétés de stabilité améliorées. Plus récemment, les documents JP 2002-128623, 2002-128628 et 2002-128629 proposent des compositions cosmétiques, notamment de maquillage, incluant à titre d'agent actif pour la brillance des esters de diacides dilinoléiques avec des dimères diols dilinoléiques.

Plus précisément, l'invention repose sur l'observation par les inventeurs qu'une composition comprenant l'association d'au moins un ester de dimère diol et d'acide avec au moins un polymère semi-cristallin particulier est brillante et de bonne tenue, en particulier non migrante.

En conséquence, la présente invention concerne, selon un de ses aspects, une composition cosmétique comprenant dans un milieu physiologiquement acceptable au moins un ester de dimère diol et d'au moins un acide mono-carboxylique en C₄ à C₃₄ ou di-carboxylique, et au moins un polymère semi-cristallin dont la température de fusion est supérieure ou égale à 30°C.

La présente invention concerne, selon un autre de ses aspects, un procédé de maquillage et/ou de soin de la peau, des lèvres et/ou des phanères comprenant l'application sur la peau, les lèvres et/ou les phanères d'au moins une composition selon l'invention.

### Ester de dimère diol et d'acide

Les esters de dimère diol et d'acide utilisables dans le cadre de la présente invention sont disponibles commercialement ou peuvent être préparés de manière conventionnelle. Ils sont notamment d'origine végétale et peuvent être obtenus par estérification d'un dimère diol avec un acide mono-carboxylique en C₄-C₃₄ comme par exemple un acide gras, ou avec un acide dicarboxylique tel qu'un dimère diacide.
Les esters de dimère diol et d'acide obtenus par estérification avec un acide mono-carboxylique peuvent avoir un poids moléculaire relativement élevé, allant d'environ 1000 à 1300 g/mol. On peut obtenir un dicarboxylate de dimère diol qui présente un poids moléculaire moyen en poids, déterminé par chromatographie de perméation de gel (GPC), allant de 2000 à 20 000 g/mol, de préférence entre 2000 et 4000 g/mol.

L'acide mono-carboxylique utilisable dans la présente invention comporte de 4 à 34 atomes de carbone, et notamment de 10 à 32 atomes de carbone.
A titre illustratif des exemples d'acide mono-carboxylique convenant à l'invention, on peut notamment citer :
- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
- les acides gras ramifiés tels que par exemple l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque, des acides gras à longue chaîne obtenus à partir de la lanoline,
- les acides gras linéaires insaturés en C₈ à C₃₄, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique,
- des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque,
- des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique, et
- des acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison et leurs mélanges.
Il s'agit plus particulièrement d' un acide gras, notamment tel que défini ci-dessus. Par acide gras, on entend un acide carboxylique obtenu par hydrolyse de graisses ou d'huiles végétales ou animales. L'acide gras peut être saturé ou insaturé.

L'ester obtenu peut être un diester, un monoester ou un de leurs mélanges. En l'occurrence, l'ester peut être un mélange de deux ou plusieurs types d'ester formés avec différents acides carboxyliques.

L'acide dicarboxylique utilisable selon l'invention doit contenir au moins deux groupes carboxyliques par molécule. Il peut notamment être représenté par la formule (I) suivante :

HOOC-(CH₂)ₙ-COOH (I)

dans laquelle n est un nombre entier de 1 à 16, de préférence de 3 à 16.
A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-ndécaméthylènedicarboxylique, l'acide 1,12-dodécaméthylène-dicarboxylique, l'acide 1,13-tridécaméthylènedicarboxylique, l'acide 1, 14-tétradécaméthylènedicarboxylique, l'acide 1,15-pentadécaméthylènedicarboxylique, l'acide 1,16-hexadécaméthylènedicarboxylique et leurs mélanges.

L'acide dicarboxylique peut également être un dimère diacide. Un dimère diacide désigne un diacide obtenu par réaction de polymérisation, notamment de dimérisation, intermoléculaire d'au moins un acide mono-carboxylique insaturé.
Ils dérivent en particulier de la dimérisation d'un acide gras insaturé notamment en C₈ à C₃₄, notamment en C₁₂ à C₂₂, en particulier en C₁₆ à C₂₀, et plus particulièrement en C₁₈.
A titre représentatif de ces acides gras insaturés, on peut notamment citer comme précédemment l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.
Selon une variante particulière, il s'agit plus particulièrement du dimère diacide dont dérive également le dimère diol à estérifier.
Plus particulièrement, il s'agit du dimère diacide obtenu par dimérisation de l'acide linoléique, éventuellement suivie d'une hydrogénation des doubles liaisons carbone carbone. Le dimère diacide peut être sous forme saturée, c'est-à-dire ne comporter aucune double liaison carbone carbone. Selon un autre mode de réalisation, les éventuelles double liaison carbone carbone du dimère diacide sont toutes ou en partie hydrogénées, après réaction d'estérification du dimère diacide avec le dimère diol.
Selon un mode de réalisation, le dimère diacide est un produit commercialisé constitué d'un acide dicarboxylique ayant environ 36 atomes de carbone. Ce produit contient également un acide trimérique et un acide monomère, dans des proportions qui dépendent du degré de pureté du produit. Classiquement, on trouve dans le commerce des produits dont la teneur en dimère diacide est supérieure à 70 % et d'autres dont la teneur en dimère diacide a été ajustée à 90 % ou plus.

On trouve également dans le commerce des dimères diacides et notamment des diacides dilinoléiques dont la stabilité vis-à-vis de l'oxydation a été améliorée par hydrogénation des doubles liaisons restant après la réaction de dimérisation.
Dans la présente invention, on peut utiliser tout dimère diacide disponible actuellement dans le commerce.
Dans une réaction d'estérification avec un acide dicarboxylique et en particulier un dimère diacide, le degré moyen d'estérification et le poids moléculaire moyen de l'ester obtenu peuvent être ajustés en faisant varier le rapport du dimère diol à l'acide dicarboxylique et en particulier au dimère diacide.
Le rapport, exprimé comme la proportion molaire d'un acide dicarboxylique basée sur le poids moléculaire moyen calculé à partir de son indice d'acide pour 1 mole de dimère diol basée sur le poids moléculaire moyen calculé à partir de son indice d'hydroxyle, est compris généralement entre 0,2 et 1,2 mole, en particulier entre 0,4 et 1,0, par exemple égal à 0,5 ou 0,7.
Au sens de la présente invention, on entend plus particulièrement désigner sous le terme « dimère diol » des diols saturés produits par hydrogénation des dimères diacides correspondants, un dimère diacide étant tel que défini ci-dessus.
En ce qui concerne le dimère diol fabriqué industriellement, il contient également généralement d'autres composants, par exemple, un trimère triol, un monoalcool et des composés de type éther, selon le degré de purification de l'acide dimérique et/ou de l'ester d'alcool inférieur de celui-ci, utilisé comme matière première. Généralement, les produits dont la teneur en dimère diol est supérieure à 70 % peuvent être utilisés dans la présente invention. Toutefois, il est préférable d'utiliser un dimère diol de grande pureté, tel qu'un composé dont la teneur en dimère diol est supérieure à 90 %.
Ainsi, un dimère diol peut être produit par hydrogénation catalytique d'un dimère diacide, lui-même obtenu par dimérisation d'au moins un acide gras insaturé notamment en C₈ à C₃₄, tels que ceux cités précédemment, notamment en C₁₂ à C₂₂, en particulier en C₁₆ à C₂₀, et plus particulièrement en C₁₈ à l'image par exemple de l'acide oléique et de l'acide linoléique.
Selon un mode de réalisation particulier, le dimère diol dérive de l'hydrogénation des fonctions acides du diacide dilinoléique.
Plus particulièrement, il s'agit du dimère diol obtenu par dimérisation de l'acide linoléique, suivie d'une hydrogénation des fonctions acides. Le dimère diol peut être sous forme saturée, c'est-à-dire ne comporter aucune double liaison carbone carbone. Selon un autre mode de réalisation, les éventuelles double liaison carbone carbone du dimère diol sont toutes ou en partie hydrogénées, après réaction d'estérification du dimère diacide avec le dimère diol.
Selon une variante de l'invention, l'ester de dimère diol est un ester de dimère diol et de dimère diacide et notamment est un composé de formule générale (II)

HO-R¹-(-OCO-R²-COO-R¹-)ₕ-OH (II)

dans laquelle :
R¹ représente un reste de dimère diol obtenu par hydrogénation du diacide dilinoléique
R² représente un reste de diacide dilinoléique hydrogéné, et
h représente un entier variant de 1 à 9.

A titre illustratif des esters convenant à l'invention, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7® .

La quantité en ester selon l'invention est généralement ajustée de manière à contrôler la brillance moyenne de la composition à la valeur souhaitée. En l'occurrence, l'ester peut être présent à raison de 1 à 99 % en particulier, notamment de 2 à 60 % en poids, en particulier de 5 à 40 % et plus particulièrement de 10 à 35 % en poids par rapport au poids total de la composition.

La composition de l'invention peut se présenter sous forme de pâte, de solide, de crème. Elle peut être une émulsion huile-dans-eau ou eau-dans-huile, un gel anhydre, solide ou souple. De préférence, elle se présente sous forme anhydre, et plus spécialement sous forme de gel anhydre, notamment coulée en stick ou en coupelle.

### Polymère semi-cristallin

Par "polymères", on entend au sens de l'invention des composés comportant au moins 2 motifs de répétition, de préférence au moins 3 motifs de répétition et plus spécialement au moins 10 motifs répétitifs.

Par "polymère semi-cristallin", on entend au sens de l'invention, des polymères comportant une partie cristallisable et une partie amorphe dans le squelette et présentant une température de changement de phase réversible du premier ordre, en particulier de fusion (transition solide-liquide). La partie cristallisable est soit une chaîne latérale (ou chaîne pendante), soit une séquence dans le squelette.

Lorsque la partie cristallisable du polymère semi-cristallin est une séquence du squelette polymérique, cette séquence cristallisable est de nature chimique différente de celle des séquences amorphes; le polymère semi-cristallin est dans ce cas un copolymère séquencé par exemple du type dibloc, tribloc ou multibloc. Lorsque la partie cristallisable est une chaîne pendante au squelette, le polymère semi cristallin peut être un homopolymère ou un copolymère.

Par "composé organique" ou "à structure organique", on entend des composés contenant des atomes de carbone et des atomes d'hydrogène et éventuellement des hétéroatomes comme S, O, N, P seuls ou en association.

La température de fusion du polymère semi-cristallin est de préférence inférieure à 150°C.

La température de fusion du polymère semi-cristallin est de préférence supérieure ou égale à 30°C et inférieure à 100°C. De préférence encore, la température de fusion du polymère semi-cristallin est de préférence supérieure ou égale à 30°C et inférieure à 60°C.

Le ou les polymères semi-cristallins selon l'invention sont des solides à température ambiante (25°C) et pression atmosphérique (760 mm de Hg), dont la température de fusion est supérieure ou égale à 30°C. Les valeurs de point de fusion correspondent au point de fusion mesuré à l'aide d'un calorimètre à balayage différentiel (D. S. C), tel que le calorimètre vendu sous la dénomination DSC 30 par la société METTLER, avec une montée en température de 5 ou 10°C par minute. (Le point de fusion considéré est le point correspondant à la température du pic le plus endotherme du thermogramme).

Le ou les polymères semi-cristallins selon l'invention ont de préférence une température de fusion supérieure à la température du support kératinique destiné à recevoir ladite composition, en particulier la peau ou les lèvres.

Le ou les polymères semi-cristallins selon l'invention peuvent être capables de structurer seuls ou en mélange, la composition sans ajout de tensioactif particulier, ni de charge, ni de cire.

Selon l'invention les polymères semi-cristallins sont avantageusement solubles dans la phase grasse, notamment à au moins 1 % en poids, à une température supérieure à leur température de fusion. En dehors des chaînes ou séquences cristallisables, les séquences des polymères sont amorphes.

Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère.

De préférence, le squelette polymérique des polymères semi-cristallins est soluble dans la phase grasse.

De préférence, les séquences ou chaînes cristallisables des polymères s emi-cristallins représentent au moins 30 % du poids total de chaque polymère et mieux au moins 40 %. Les polymères semi-cristallins à chaînes latérales cristallisables sont des homo ou des copolymères. Les polymères semi-cristallins de l'invention à séquences cristallisables sont des copolymères, séquencés ou multiséquencés. Ils peuvent être obtenus par polymérisation de monomère à double liaisons réactives (ou éthyléniques) ou par polycondensation. Lorsque les polymères de l'invention sont des polymères à chaînes latérales cristallisables, ces derniers sont avantageusement sous forme aléatoire ou statistique.

De préférence, les polymères semi-cristallins de l'invention sont d'origine synthétique. Selon un mode de réalisation de l'invention, les polymères semi-cristallins de l'invention ne comportent pas de squelette polysaccharidique.

Les polymères semi-cristallins utilisables dans l'invention peuvent être choisis en particulier parmi:
- les copolymères séquencés de polyoléfines à cristallisation contrôlée, dont les monomères sont décrits dans EP-A-0 951 897.
- les polycondensats et notamment de type polyester, aliphatique ou aromatique ou aliphatique/aromatique,
- les homo- ou co-polymères portant au moins une chaîne latérale cristallisable et les homo- ou co-polymères portant dans le squelette au moins une séquence cristallisable, comme ceux décrits dans le document US-A-5 156 911,
- les homo- ou co-polymères portant au moins une chaîne latérale cristallisable en particulier à groupement(s) fluoré(s), tels que décrits dans le document WO-A-01/19333,
- et leurs mélanges.

Dans les deux derniers cas, la ou les chaînes latérales ou séquences cristallisables sont hydrophobes.

### A) Polymères semi-cristallins à chaînes latérales cristallisables

On peut citer en particulier ceux définis dans les documents US-A-5 156 911 et WO-A-01/19333.
. Ce sont des homopolymères ou copolymères comportant de 50 à 100 % en poids de motifs résultant de la polymérisation de un ou plusieurs monomères porteurs de chaîne latérale hydrophobe cristallisable.
. Ces homo- ou co-polymères sont de toute nature du moment qu'ils présentent les conditions indiquées ci-après avec en particulier la caractéristique d'être solubles ou dispersables dans la phase grasse, par chauffage au-dessus de leur température de fusion Pf. Ils peuvent résulter :
   de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique.
   - de la polycondensation d'un ou plusieurs monomères porteurs de groupes co-réactifs (acide carboxylique ou sulfonique, alcool, amine ou isocyanate), comme par exemple les polyesters, les polyuréthanes, les polyéthers, les polyurées, les polyamides.

D'une façon générale les motifs (chaînes ou séquences) cristallisables des polymères semi-cristallins selon l'invention, proviennent de monomère(s) à séquence(s) ou chaîne(s) cristallisable(s), utilisé(s) pour la fabrication des polymères semi-cristallins. Ces polymères sont choisis notamment parmi les homopolymères et copolymères résultant de la polymérisation d'au moins un monomère à chaîne(s) cristallisable(s) qui peut être représenté par la formule X : 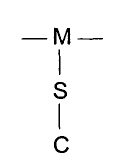 avec M représentant un atome du squelette polymérique
S représentant un espaceur
C représentant un groupe cristallisable

Les chaînes « -S-C » cristallisables peuvent être aliphatiques ou aromatiques, éventuellement fluorées ou perfluorées. « S » représente notamment un groupe (CH₂)ₙ ou (CH₂CH₂O)ₙ ou (CH₂O), linéaire ou ramifié ou cyclique, avec n entier allant de 0 à 22. De préférence « S » est un groupe linéaire. De préférence, « S » et « C » sont différents.

Lorsque les chaînes cristallisables sont des chaînes aliphatiques hydrocarbonées, elles comportent des chaînes alkyle hydrocarbonées à au moins 11 atomes de carbone et au plus 40 atomes de carbone et mieux au plus 24 atomes de carbone. Il s'agit notamment de chaînes aliphatiques ou chaînes alkyle possédant au moins 12 atomes de carbone et de préférence, il s'agit de chaînes alkyle en C₁₄-C₂₄. de préférence en C₁₆-C₂₂ .Lorsqu'il s'agit de chaînes alkyle fluorées ou perfluorées, elles comportent au moins 11 atomes de carbone dont au moins 6 atomes de carbone sont fluorés.

Comme exemple d'homopolymères ou de copolymères semi-cristallins à chaîne(s) cristallisable(s), on peut citer ceux résultant de la polymérisation d'un ou plusieurs monomères suivants : les (méth)acrylates d'alkyle saturés avec le groupe alkyle en C₁₄-C₂₄, les (méth)acrylates de perfluoroalkyle avec un groupe alkyle perfluoro en C₁₁-C₁₅, les N-alkyl (méth)acrylamides avec le groupe alkyle en C₁₄ à C₂₄ avec ou sans atome de fluor, les esters vinyliques à chaînes alkyle ou perfluoro (alkyle) avec le groupe alkyle en C₁₄ à C₂₄ (avec au moins 6 atomes de fluor pour une chaîne perfluoro alkyle), les éthers vinyliques à chaînes alkyle ou perfluoro (alkyle) avec le groupe alkyle en C₁₄ à C₂₄ et au moins 6 atomes de fluor pour une chaîne perfluoro alkyle, les alpha-oléfines en C₁₄ à C₂₄ comme par exemple l'octadécène, les para-alkyl styrènes avec un groupe alkyle comportant de 12 à 24 atomes de carbone, leurs mélanges.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un di-isocyanate.

Lorsque les polymères objets de l'invention sont des copolymères, ils contiennent, en plus, de 0 à 50 % de groupes Y ou Z résultant de la copolymérisation :
α) de Y qui est un monomère polaire ou non polaire ou un mélange des deux :
   . Lorsque Y est un monomère polaire, c'est soit un monomère porteur de groupes polyoxyalkylénés (notamment oxyéthyléné et/ou oxypropyléné), un (méth)acrylate d'hydroxyalkyle comme l'acrylate d'hydroxyéthyle, le (méth)acrylamide, un N-alkyl(méth)acrylamide, un NN-dialkyl(méth)acrylamide comme par exemple le NN-diisopropylacrylamide ou la N-vinyl-pyrolidone (NVP), le N-vinyl caprolactame, un monomère porteur d'au moins un groupe acide carboxylique comme les acides (méth)acryliques, crotonique, itaconique, maléique, fumarique ou porteur d'un groupe anhydride d'acide carboxylique comme l'anhydre maléique, et leurs mélanges.
   . Lorsque Y est un monomère non polaire il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle en C₁ à C₁₀, comme l'α-méthylstyrène, un macromonomère du type polyorganosiloxane à insaturation vinylique.

   Par "alkyle", on entend au sens au sens de l'invention un groupement saturé notamment en C₈ à C₂₄, sauf mention exprès.
β) de Z qui est un monomère polaire ou un mélange de monomères polaires. Dans ce cas, Z a la même définition que le "Y polaire" défini ci-dessus.

De préférence, les polymères semi-cristallins à chaîne latérale cristallisable sont des homopolymères d'alkyl(méth)acrylate ou d'alkyl(méth)acrylamide avec un groupe alkyle tel que défini ci-dessus, et notamment en C₁₄-C₂₄, notamment en C₁₆-C₂₀, des copolymères de ces monomères avec un monomère hydrophile de préférence de nature différente de l'acide (méth)acrylique comme la N-vinylpyrrolidone ou l'hydroxyéthyl (méth)acrylate et leurs mélanges.

De façon avantageuse, le ou les polymères semi-cristallins à chaîne latérale cristallisable ont une masse moléculaire moyenne en poids Mp allant de 5 000 à 1 000 000, de préférence de 10 000 à 800 000, préférentiellement de 15 000 à 500 000, de préférence encore de 100 000 à 200 000.

A titre d'exemple particulier de polymère semi-cristallin utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables et présentent la formule X précédente.

Par exemple, on choisit le produit Intelimer® IPA 13-1 de la société Landec, qui est un polyacrylate de stéaryle de poids moléculaire d'environ 145 000 et dont la température de fusion est égale à 49°C.

Les polymères semi-cristallins peuvent être notamment ceux décrits dans les exemples 3, 4, 5, 7, 9 du brevet US-A-5 156 911 à groupement -COOH, résultant de la copolymérisation d'acide acrylique et d'alkyl(méth)acrylate en C₅ à C₁₆ de température de fusion allant de 20°C à 35°C et plus particulièrement de la copolymérisation :
. d'acide acrylique, d'hexadécylacrylate et d'isodécylacrylate dans un rapport 1/16/3,
. d'acide acrylique et de pentadécylacrylate dans un rapport 1/19,
. d'acide acrylique, d'hexadécylacrylate, éthylacrylate dans un rapport 2,5/76,5/20,
. d'acide acrylique, d'hexadécylacrylate et de méthylacrylate dans un rapport 5/85/10,
. d'acide acrylique, d'octadécylméthacrylate dans un rapport 2,5/97,5.

On peut aussi utiliser le polymère Structure « O » de National Starch tel que celui décrit dans le document US-A-5 736 125 de température de fusion de 44°C

Les polymères semi-cristallins peuvent être notamment les polymères semi-cristallins à chaînes pendantes cristallisables comportant des groupements fluorés tels que décrits dans les exemples 1, 4, 6, 7 et 8 du document WO-A-01/19333.

On peut encore utiliser les polymères semi-cristallins obtenus par copolymérisation d'acrylate de stéaryle et d'acide acrylique ou de NVP tels que décrits dans le document US-A-5 519 063 ou EP-A- 550 745.

On peut aussi utiliser les polymères semi-cristallins obtenus par copolymérisation de l'acrylate de béhényle et de l'acide acrylique ou de NVP, tels que décrits dans les documents US-A-5 519 063 et EP-A- 055 0745 .

### B) Les polymères portant dans le squelette au moins une séquence cristallisable

Il s'agit encore de polymères solubles ou dispersables dans la phase grasse par chauffage au-dessus de leur point de fusion Pf. Ces polymères sont notamment des copolymères séquencés constitués d'au moins deux séquences de nature chimique différente dont l'une est cristallisable.

Le polymère portant dans le squelette au moins une séquence cristallisable peut être choisi parmi les copolymères séquencés d'oléfine ou de cyclooléfine à chaîne cristallisable comme ceux issus de la polymérisation séquencée de :
- cyclobutène, cyclohexène, cyclooctène, norbornène (c'est-à-dire bicyclo(2,2,1)heptène-2), 5-méthylnorbornène, 5-éthylnorbornène, 5,6-diméthylnorbornène, 5,5,6-triméthyl norbornène, 5-éthylidène-norbornène, 5-phényl-norbonène, 5-benzylnorbornène, 5-vinyl norbornène, 1,4,5,8-diméthano-1,2,3,4,4a,5,8a-octahydronaphtalène, dicyclopentadiène ou leurs mélanges, avec
- l'éthylène, le propylène, le 1-butène, le 3-méthyl-1-butène, le 1-hexène, le 4-méthyl-1-pentène, le 1-octène, le 1-décène, le 1-éicosène ou leurs mélanges,
et en particulier les copoly(éthylène/norbornène) blocs et les terpolymères (éthylène/propylène/éthylidène-norbornène), blocs. On peut aussi utiliser ceux résultants de la copolymérisation séquencée d'au moins 2 α-oléfines en C₂-C₁₆ et mieux en C₂-C₁₂ tels que ceux cités précédemment et en particulier les bipolymères séquencés d'éthylène et d'1-octène.

Le polymère portant dans le squelette au moins une séquence cristallisable peut être choisi parmi les copolymères présentant au moins une séquence cristallisable, le reste du copolymère étant amorphe (à température ambiante). Ces copolymères peuvent, en outre, présenter deux séquences cristallisables de nature chimique différente.

Les copolymères préférés sont ceux qui possèdent à la fois à température ambiante, une séquence cristallisable et une séquence amorphe à la fois hydrophobe et lipophile réparties séquentiellement. On peut citer par exemple les polymères possédant une des séquences cristallisables et une des séquences amorphes suivantes :
- Séquence cristallisable par nature de type polyester comme les poly(alkylène téréphtalate), ou de type polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :
α) les copolymères séquencés poly(ε-caprolactone)-b-poly(butadiène), utilisés de préférence hydrogénés, tels que ceux décrits dans l'article "Melting behavior of poly(-caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene-(ethylene-alt-propylene)" de P. Rangarajan et al., Macromolecules, 26, 4640-4645 (1993) et "Polymer agregates with crystalline cores : the system poly(ethylene)-poly(ethylene-propylene)" P. Richter et al., Macromolécules, 30, 1053-1068 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

### C) Polycondensats de type polyester, aliphatique ou aromatique ou aliphatique/aromatique

Les polycondensats polyester peuvent être choisis parmi les polyesters aliphatiques. Leur masse moléculaire est de préférence supérieure ou égale à 200 et inférieure ou égal à 10000, et de préférence encore supérieure ou égale à 300 et inférieure ou égal à 5000, de préférence supérieure ou égale à 500 et inférieure ou égale à 2 000 g/mol.

Les polycondensats polyester sont en particulier choisis parmi les polycaprolactones. En particulier, les polycaprolactones peuvent être choisies parmi les homopolymères d'ε-caprolactones. L'homopolymérisation peut être initiée avec un diol, notamment un diol ayant de 2 à 10 atomes, tels que le diéthylène glycol, le 1,4-butanediol, le néopentyl glycol.

On peut utiliser par exemple les polycaprolactones, notamment celles commercialisées sous le nom de CAPA ® 240 (point de fusion de 68°C et poids moléculaire de 4000), 223 (point de fusion de 48°C et poids moléculaire de 2000), 222 (point de fusion de 48°C et poids moléculaire de 2000), 217 (point de fusion de 44°C et poids moléculaire de 1250), 2125 (point de fusion de 45°C et poids moléculaire de 1 250), 212 (point de fusion de 45°C et poids moléculaire de 1000), 210 (point de fusion de 38°C et poids moléculaire de 1000), 205 (point de fusion de 39°C et poids moléculaire de 830) par la société SOLVAY, PCL-300, PCL-700 par la société UNION CARBIDE.

On peut utiliser en particulier la CAPA ® 2125 dont la température de fusion est comprise entre 35 et 45°C et dont la masse moléculaire en poids est égale à 1250.

Les polymères semi-cristallins de la composition de l'invention peuvent être ou non réticulés en partie du moment où le taux de réticulation ne gène pas leur dissolution ou dispersion dans la phase grasse par chauffage au-dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère comme par exemple les interactions dipolaires entre ionomères carboxylates, ces interactions étant en faible quantité et portées par le squelette du polymère ; soit à une séparation de phase entre les séquences cristallisables et les séquences amorphes, portées par le polymère.
De préférence, les polymères semi-cristallins de la composition selon l'invention sont non réticulés.

En pratique, la quantité totale de polymère(s) semi-cristallin(s) représente de 0,1 à 80 % du poids total de la composition et mieux de 0,5 à 40 % et encore mieux de 3 à 30 %. De préférence, il représente de 5 % à 25% en poids de la composition.

### Phase aqueuse

La composition selon l'invention peut comprendre au moins un milieu aqueux, constituant une phase aqueuse, qui peut former la phase continue de la composition.
La phase aqueuse peut être constituée essentiellement d'eau.
Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.
La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids par rapport au poids total de la composition.
Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.
Un tel milieu peut également comprendre au moins une huile volatile telle que définie ci-après.

### Phase grasse

La composition, notamment lorsqu'elle est destinée à être appliquée sur les lèvres, peut comporter au moins une phase grasse et notamment au moins un corps gras liquide à température ambiante (25°C) et à pression atmosphérique et/ou un corps gras solide à température ambiante et à pression atmosphérique tel que les cires, les gommes et leurs mélanges. La phase grasse peut en outre contenir des agents gélifiants et structurants d'huiles de nature organique et/ou des solvants organiques lipophiles.
Selon une variante particulière de l'invention, la composition cosmétique est exempte de paraffine, de vaseline et de lanoline. En effet, les lanolines présentent l'inconvénient d'être sensibles à la chaleur et aux ultraviolets, et ont tendance à s'oxyder dans le temps avec un dégagement d'odeur désagréable, ce qui limite leur utilisation dans les compositions cosmétiques. De plus, lorsque les lanolines sont associées avec des huiles couramment utilisées dans le domaine cosmétique, les compositions obtenues présentent des problèmes de collant, qui sont d'autant plus prononcés que l'huile utilisée possède une viscosité élevée.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).
Par "huile non volatile", on entend une huile restant sur la peau à température ambiante et pression atmosphérique au moins plusieurs heures et ayant notamment une pression de vapeur inférieure à 0,01 mm de Hg (1,33 Pa).
Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore. Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'iso-hexadécane, et par exemple les huiles vendues sous les noms commerciaux d'Isopars® ou de Permetyls , les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.
Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.
L'huile volatile peut être présente dans la composition selon l'invention à une teneur allant de 0,1 % à 98 % en poids, notamment de 1 % à 65 % en poids, et en particulier de 2 % à 50 % en poids, par rapport au poids total de la composition.
Les huiles non volatiles peuvent notamment être choisies parmi les huiles hydrocarbonées le cas échéant fluorées et/ou les huiles siliconées non volatiles.
Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810® , 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.
   Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsi-loxanes, les diphényl diméthicones, les diphényl méthyldiphényl trisiloxanes, et les 2-phényléthyl triméthylsiloxysilicates et leurs mélanges.
   Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.
   Les huiles peuvent représenter de 0,01 à 99 % du poids total de la composition, de préférence de 0,05 à 60 % et mieux de 1 à 35 %.

En ce qui concerne le corps gras solide à température ambiante et à pression atmosphérique, il peut être choisi parmi les cires, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

Les cires peuvent être hydrocarbonées, fluorées et/ou siliconées et être d'origine végétale, minérale, animale et/ou synthétique. En particulier, les cires présentent une température de fusion supérieure à 45 °C.
Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La quantité de toutes les cire(s) contenues dans la composition va avantageusement de 15% à 35%, de préférence de 20% à 30% % en poids du poids total de la composition.

La composition selon l'invention peut également contenir un composé pâteux.

La composition peut comprendre des gommes. Les gommes utilisables dans l'invention se présentent généralement sous forme solubilisée dans une huile,
Par gomme on entend un corps gras qui se présente sous forme de polymère ayant un poids moléculaire moyen en poids de 50 000 à 1 000 000. La gomme est souvent vendue en dispersion dans un solvant organique, du type huile de silicone.
La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

### Charges et pigments

Au sens de l'invention, on entend désigner sous le terme « charges » tout composé, organique et/ou inorganique introduit dans la composition cosmétique afin d'ajuster ses propriétés en terme de texture ou en d'autres termes pour contrôler ses propriétés rhéologiques. Les pigments et nacres sont notamment exclus de cette définition.
Selon une variante particulière de l'invention, les compositions cosmétiques comprennent moins de 15% en poids, en particulier moins de 10% en poids, notamment moins de 7% en poids de charge par rapport au poids total de la composition.
Il peut notamment s'agir de charges sphériques comme par exemple le talc, le stéarate de zinc, le mica, le kaolin, les poudres de polyamide (Nylon® ) (Orgasol® de chez Atochem), les poudres de polyéthylène, les poudres de polymères de tétrafluoroéthylène (Téflon® ), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning), les microbilles de résine de silicone (Tospearls® de Toshiba, par exemple), et les organopolysiloxanes élastomères.

La composition selon l'invention peut en outre contenir des agents tensioactifs émulsionnants présents notamment en une proportion allant de 0,1 à 30 % en poids, et mieux de 5 % à 15 % en poids, par rapport au poids total de la composition.
Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.
Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.
On utilise de préférence des tensioactifs permettant l'obtention d'émulsion huile-dans-eau ou cire-dans-eau.

La composition de l'invention, comprend généralement au moins un agent de coloration pouvant notamment être présent à raison de 0,01 % à 40 % en poids, notamment de 0,01 % à 30 % en poids et en particulier de 0,05 % à 25 % en poids, par rapport au poids total de la composition.
Ces ou cet agent de coloration peuvent être choisis parmi les pigments, les colorants hydrosolubles ou liposolubles, les nacres et leurs mélanges.
Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.
Les pigments peuvent être présents dans la composition à raison de 0,01 % à 25 % en poids, en particulier de 0,01 % à 15 % en poids, et notamment de 0,02 % à 5 % en poids par rapport au poids de la composition.
Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en nanotubes présente dans la composition cosmétique considérée.
Les nacres peuvent être présentes dans la composition à raison de 0,01 % à 25 % en poids, notamment de 0,01 % à 15 % en poids, et en particulier de 0,02 % à 5 % en poids, par rapport au poids total de la composition.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
La composition peut comprendre également des colorants hydrosolubles ou liposolubles en une teneur allant de 0,01 % à 6 % en poids, par rapport au poids total de la composition, notamment allant de 0,01 % à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

### Additifs

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.
Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.
La composition de l'invention peut être obtenue selon les procédés de préparation classiquement utilisés en cosmétique ou en dermatologie.
La composition de l'invention peut se présenter sous la forme de composition solide, compactée ou coulée en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.
En l'occurrence, elle peut se présenter sous la forme de rouges à lèvres, baumes à lèvres, fonds de teint coulés, produits anti-cernes, produits « correcteurs » ou « embellisseurs » de teint, et/ou fards à paupières ou à joues.

Elle peut toutefois se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. Selon une variante particulière, elle se présente sous la forme d'une émulsion.
La composition selon l'invention peut se présenter sous la forme d'une composition, colorée ou non, sous forme d'une composition de protection solaire ou de démaquillage ou encore sous forme d'une composition hygiénique. Elle contient notamment des actifs cosmétiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B3, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être cosmétiquement acceptable, à savoir non toxique et susceptible d'être appliquée sur la peau, les phanères ou les lèvres d'êtres humains.

Les exemples de compositions ci-après sont donnés à titre illustratif et sans caractère limitatif.

### Exemples 1 : Rouge à lèvres

| **Nom chimique** | **Exemple 1** |
|---|---|
| Dispersion de polymère | 30 |
| Triglycéride d'acide 2-décyl tétradécanoïque | 2.02 |
| Copolymères dimères dilinoleyl diols / dimères dilinoléiques (LUSPLAN DD-DA 5) | 10 |
| Octyldodécanol | 9 |
| Conservateurs | 0.47 |
| Polycaprolactone de PM 1250 g/mol (CAPA 1215 de SOLVAY) | 9 |
| Polylaurate de vinyle | - |
| Copolymère vinyl pyrrolidone / eicosène | 6 |
| Cire microcristalline | 10 |
| Cire de polyéthylène | 2 |
| Cire de polyméthylène de PF 40°C | 10 |
| Pigments | 6.03 |
| Silice enrobée dimethicone | 5 |
| Parfum | 0.48 |
| TOTAL | 100 |

### Synthèse de la dispersion de particules de polymère :

On prépare une dispersion de copolymère non réticulé d'acrylate de méthyle et d'acide acrylique dans un rapport 85/15, dans l'heptane, selon la méthode de l'exemple 1 du document EP-A-749 746. Lorsque la polymérisation est terminée, on ajoute du polyisobutène hydrogéné et on distille l'heptane sous vide.
On obtient ainsi une dispersion de particules de poly(acrylate de méthyle/acide acrylique) stabilisées dans le polyisobutène hydrogéné par un copolymère dibloc séquencé polystyrène/copoly(éhylène-propylène) vendu sous le nom de Kraton G1701, ayant un taux de matière sèche de 21% en poids, et une taille moyenne de particule égale à 150 nm.

### Mode opératoire de préparation du rouge à lèvres

Toutes les matières premières sont pesées dans un poêlon à double paroi à circulation d'huile, puis mises à chauffer sous agitation (turbine).
Après fonte totale des matières et homogénéisation du mélange, celui-ci est broyé 5 fois de suite à la broyeuse tricylindre. La pâte obtenue est mise à stabiliser pendant 24 heures à 20°C puis conditionnée dans des bouillottes.

### Evaluation (in vitro) :

La formule a été testée in vitro selon le test décrit précédemment consistant à évaluer la résistance de la formule à l'eau et à l'huile. Les résultats sont les suivants :

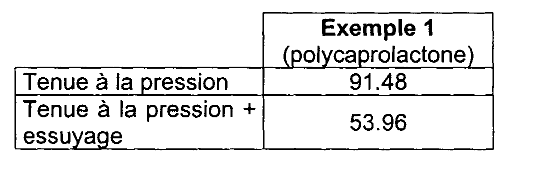

### Exemple 2 : rouge à lèvres

| | Pourcentages massiques |
|---|---|
| Triisocetyl citrate | 9.78 |
| Octyldodécanol stéarate d'acide poly(12-hydroxystéarique) | 15.59 |
| Solsperse 21 000 par la société Avecia | 1.90 |
| Isononyl isononanoate | 11.12 |
| Isopropyl isostéarate | 19;00 |
| Squalane | 3.00 |
| Copolymères dimères dilinoleyl diols / dimères dilinoléiques (LUSPLAN DD-DA 5) | 6.00 |
| Pigments | 7.3 |
| Kaolin | 3.30 |
| Polyméthacrylate de méthyle | 0.50 |
| Nylon | 2 |
| Hydrogenated coco-glycerides Polyacrylate de stéaryle (Intelimer® IPA 13-1 | 2 |
| de la société Landec) | 7.50 |
| Polyacrylate de béhényle | 11 |

### Préparation du polyacrylate de béhényle

Dans un réacteur d'1l muni d'une agitation centrale avec ancre, d'un réfrigérant et d'un thermomètre, on introduit 120g de polyisobutène hydrogéné que l'on chauffe de la température ambiante à 80°C en 45 min. A 80°C, on introduit en 2h le mélange C₁ suivant :
40g de cyclohexane + 4g de Trigonox 141 [2,5 bis (2-éthyl hexanoyl peroxy) - 2,5 - diméthyl hexane].
30 min après le début de la coulée du mélange C₁, on introduit en 1h30 le mélange C₂ constitué de :
200 g d'acrylate de béhényle + 400 g de cyclohexane.
A la fin des deux coulées, on laisse agir 3h supplémentaires à 80°C puis on distille à pression atmosphérique la totalité du cyclohexane présent dans le milieu réactionnel.
On obtient alors le polymère à 60 % en poids en matière active dans le Parléam.

Le polymère obtenu est à 60% en poids en matière active dans le Parléam. Sa masse moléculaire moyenne en poids est de 17 000-27 000 et sa T_{f} de 58°C.

## Revendications

1. Composition cosmétique comprenant au moins un ester de dimère diol et d'au moins un acide mono-carboxylique en C₄ à C₃₄ ou di-carboxylique, et au moins un polymère semi-cristallin à structure organique dont la température de fusion est supérieure ou égale à 30°C.

2. Composition selon la revendication 1, **caractérisée en ce** le polymère semi-cristallin est choisi parmi les homopolymères et copolymères comportant des motifs résultant de la polymérisation de un ou plusieurs monomères porteurs de chaîne(s) latérale(s) hydrophobe(s) cristallisable(s).

3. Composition selon l'une des revendications précédentes, **caractérisée en ce que** le polymère semi-cristallin est choisi parmi les homopolymères et copolymères résultant de la polymérisation d'au moins un monomère à chaîne(s) cristallisable(s) de formule X : 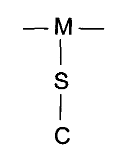
avec M représentant un atome du squelette polymérique
S représentant un espaceur
C représentant un groupe cristallisable
et leurs mélanges, avec « S-C » représentant une chaîne alkyle à au moins 11 atomes de carbone, éventuellement fluorée ou perfluorée.

4. Composition selon l'une des revendications précédentes, **caractérisée en ce que** le polymère semi-cristallin est choisi parmi les polymères résultant de la polymérisation d'au moins un monomère choisi parmi l'acide acrylique, méthacrylique, crotonique, itaconique, maléique, l'anhydride maléique et leurs mélanges.

5. Composition selon l'une des revendications précédentes, **caractérisée en ce que** le polymère semi-cristallin est choisi parmi les homopolymères et copolymères résultant de la polymérisation d'au moins un monomère à chaîne cristallisable choisis parmi les (méth)acrylates d'alkyle saturés en C₁₄-C₂₄, les (méth)acrylates de perfluoroalkyle en C₁₁-C₁₅, les N alkyl (méth)acrylamides en C₁₄ à C₂₄ avec ou sans atome de fluor, les esters vinyliques à chaînes alkyle ou perfluoroalkyle en C₁₄ à C₂₄, les éthers vinyliques à chaînes alkyle ou perfluoroalkyle en C₁₄ à C₂₄, les alpha-oléfines en C₁₄ à C₂₄, les para-alkyl styrènes avec un groupe alkyle comportant de 12 à 24 atomes de carbone, leurs mélanges.

6. Composition selon l'une des revendications précédentes, **caractérisée en ce que** le polymère semi-cristallin est choisi parmi des homopolymères d'alkyl(méth)acrylate ou d'alkyl(méth)acrylamide tels que le groupe alkyle est en C₁₄ à C₂₄.

7. Composition selon la revendication précédente, **caractérisée en ce que** le polymère semi-cristallin est choisi parmi les homopolymères d'alkyl(méth)acrylate tels que le groupe alkyle est en C₁₆ à C₂₀.

8. Composition selon l'une des revendications 2 à 7, **caractérisée en ce que** le polymère semi-cristallin a une masse moléculaire moyenne en poids allant de 5 000 à 1 000 000, de préférence de 10 000 à 800 000, préférentiellement de 15 000 à 500 000.

9. Composition selon la revendication 1, **caractérisée en ce que** le polymère semi-cristallin est un polycondensat polyester.

10. Composition selon la revendication 9, **caractérisée en ce que** le polycondensat polyester est choisi parmi les polycaprolactones.

11. Composition selon l'une des revendications 8 à 10, **caractérisée en ce que** le polycondensat polyester a une masse moléculaire supérieure ou égale à 200 et inférieure ou égal à 10000, de préférence supérieure ou égale à 300 et inférieure ou égal à 5000, et de préférence encore supérieure ou égale à 500 et inférieure ou égal à 2000.

12. Composition selon l'une des revendications 8 à 11, **caractérisée en ce que** le polymère semi-cristallin a une température de fusion supérieure ou égale à 30°C et inférieure ou égale à 100°C, de préférence supérieure ou égale à 30°C et inférieure ou égale à 60°C.

13. Composition selon l'une des revendications précédentes, **caractérisée en ce que** le polymère semi-cristallin représente de 0,1 à 80 % du poids total de la composition et mieux de 0,5 à 40 % et encore mieux de 3 à 30 %, de préférence de 5 % à 25% en poids de la composition.

14. Composition selon l'une des revendications précédentes, **caractérisée en ce que** l'acide mono-carboxylique est choisi parmi
- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
- les acides gras,
- des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque et
- des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique
- et leurs mélanges.

15. Composition selon la revendication 14, **caractérisée en ce que** l'acide gras est choisi parmi :
- des acides gras ramifiés tels que l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque,
- des acides gras linéaires insaturés en C₈ à C₃₄, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et
- des acides gras d'origine naturelle, tels que les acides g ras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison
- et leurs mélanges.

16. Composition selon l'une des revendications précédentes, **caractérisée en ce que** l'acide dicarboxylique est choisi parmi
- les composés de formule (I) HOOC-(CH₂)ₙ-COOH dans laquelle n est un nombre entier de 1 à 16, de préférence de 3 à 16, et
- les dimères diacides obtenus par dimérisation d'au moins un acide mono-carboxylique insaturé.

17. Composition selon la revendication 16, **caractérisée en ce que** l'acide dicarboxylique est un dimère diacide .

18. Composition selon la revendication 16, **caractérisée en ce que** l'acide mono-carboxylique insaturé est un acide gras insaturé en C₈ à C₃₄, notamment en C₁₂ à C₂₂, en particulier en C₁₆ à C₂₀ et plus particulièrement en C₁₈.

19. Composition selon la revendication 16, **caractérisée en ce que** l'acide mono-carboxylique insaturé est choisi parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

20. Composition selon l'une quelconque des revendications 16 à 18, **caractérisée en ce que** le dimère diacide est le diacide dilinoléique.

21. Composition selon la revendication 19 ou 20, **caractérisée en ce que** le dimère diacide est saturé.

22. Composition selon l'une quelconque des revendications précédentes, **caractérisée en ce que** le dimère diol dérive de l'hydrogénation d'un dimère diacide.

23. Composition selon la revendication 22, **caractérisée en ce que** le dimère diacide dérive de la dimérisation d'un acide gras insaturé, notamment en C₈ à C₃₄ et en particulier en C₁₂ à C₂₂, en particulier en C₁₆ à C₂₀, et plus particulièrement en C₁₈.

24. Composition selon la revendication précédente, **caractérisée en ce que** l'acide gras insaturé est tel que défini en revendication 18.

25. Composition selon l'une quelconque des revendications 22 à 24, **caractérisée en ce que** le dimère diol dérive de l'hydrogénation du diacide dilinoléique.

26. Composition selon l'une quelconque des revendications 22 à 25, **caractérisée en ce que** le dimère diol est saturé.

27. Composition selon l'une quelconque des revendications 24 à 26, **caractérisée en ce que** le dimère diacide est identique au dimère diacide dont dérive le dimère diol.

28. Composition selon les revendications 22 et 27, **caractérisée en ce que** l'ester est un composé de formule générale (II) :
HO-R¹-(-OCO-R²-COO-R¹-)ₕ-OH (II)
dans laquelle :
R¹ représente un reste de dimère diol obtenu par hydrogénation du diacide dilinoléique,
R² représente un reste de diacide dilinoléique hydrogéné, et
h représente un entier variant de 1 à 9.

29. Composition selon l'une quelconque des revendications précédentes, **caractérisée en ce que** l'ester est présent à raison de 1 à 99 % en poids, notamment de 2 à 60 % en poids, en particulier de 5 à 40 % en poids et plus particulièrement de 10 à 35 % en poids par rapport au poids total de la composition.

30. Composition selon l'une des revendications précédentes, se présentant sous la forme d'un produit de soin et/ou de maquillage de la peau et/ou des lèvres.

31. Composition selon l'une des revendications précédentes, se présentant sous forme d'un fond de teint, d'un fard à joues ou à paupières, d'un rouge à lèvres, d'une base ou baume de soin pour les lèvres, d'un produit anti-cernes, d'un eye liner, d'un mascara.

32. Procédé de maquillage et/ou de soin de la peau, des lèvres et/ou des phanères comprenant l'application sur la peau, les lèvres et/ou les phanères d'au moins une composition selon l'une quelconque des revendications 1 à 31.
